# EUROPÄISCHE PATENTSCHRIFT

(11) **EP 4 320 133 B1**
(45) Veröffentlichungstag und Bekanntmachung des Hinweises auf die Patenterteilung: **28.08.2024**
(21) Anmeldenummer: 21717081.0
(22) Anmeldetag: 07.04.2021
(51) Int. Cl.: C07F 7/08

(54) **VERFAHREN ZUR UMSETZUNG VON SILICON MIT HYDROTHERMALEM ODER ÜBERKRITISCHEM WASSER**
PROCESS OF REACTING SILICONE WITH HYDROTHERMAL OR SUPERCRITICAL WATER
PROCÉDÉ DE RÉACTION DE SILICONE AVEC DE L'EAU HYDROTHERMALE OU SUPERCRITIQUE

(43) Veröffentlichungstag der Anmeldung: 14.02.2024
(73) Patentinhaber: Wacker Chemie AG, 81671 München (DE)
(72) Erfinder: ULBRICH, Markus, 84489 Burghausen (DE); TILLMANN, Jan, 80798 München (DE); VOIT, Harald, 84571 Reischach (DE)
(86) Internationale Anmeldenummer: PCT/EP2021/059103
(87) Internationale Veröffentlichungsnummer: WO 2022/214176

(56) Entgegenhaltungen:
- CN-A- 111 498 854

## Beschreibung

Die Erfindung betrifft ein Verfahren zur Umsetzung von Silicon mit hydrothermalem oder überkritischem Wasser.

Siliconabfälle werden aktuell der Verbrennung zugeführt. Sie sind schlecht brennbar, belasten die Verbrennung durch Staubbildung und benötigen hohe Luftströme/Brennraumtemperaturen zur Umsetzung. Der Staub fällt hierbei zusammen mit der Asche als giftiges Nebenprodukt an und muss aufwändig entsorgt werden.

Der Einsatz von hydrothermalem Wasser als chemisches Reagenz wurde 1913 von Friedrich Bergius im Zuge der Kohlevergasung untersucht und dabei als Schlüsselkomponente für die natürliche Inkohlung identifiziert. Im hydrothermalen Bereich sinkt die Dielektrizitätskonstante des Wassers so weit, dass unpolare Stoffe gelöst werden können. Durch die hohe Temperatur und den hohen Druck läuft dabei für Lignozellulose, also organische Substanz, ein komplexes Reaktionsnetzwerk ab. Die Lignozellulose wird dabei durch Hydrolyse von C-O Bindungen und Feststoffreaktionen bzw. intramolekolare Umlagerungen und Eliminationsreaktionen des Lignins abgebaut und in einen C-reichen Stoff umgewandelt. Je nach Temperatur wird ein Feststoff, ein Öl oder Gas erhalten. Die hydrothermalen Prozesse sind im Bereich der Biomassenutzung in den letzten Jahren stark in den Fokus der Forschung gerückt.

Kenichiro Itami, Koji Terakawa, Jun-ichi Yoshida, and Okitsugu Kajimoto; "The Carbon-Silicon Bond Cleavage of Organosilicon Compounds in Supercritical Water" The Chemical Society of Japan Bull. Chem. Soc. Jpn., 77, 2071-2080 (2004) 2071 beschreibt die Spaltung von Si-C Bindungen von Silanen mit überkritischem Wasser. CN 111498854A offenbart ein Verfahren, bei dem Silicon mit überkritischem Wasser behandelt wird, um eine Substanz das Silicium enthält mit SiO₂-Bindungen herzustellen.

Gegenstand der Erfindung ist ein Verfahren, bei dem Silicon mit hydrothermalem Wasser ab Temperaturen von 140°C oder überkritischem Wasser umgesetzt wird zu einem Feststoffgemisch, das Silicium enthält,bei dem der Begriff Silicon oligomere oder polymere Organosiloxane umfasst, in denen Siliciumatome über Sauerstoffatome verbunden sind und in denen mindestens ein Teil der Siliciumatome einen oder mehrere organische Substituenten tragen, sowie Organosiloxane enthaltende Zusammensetzungen.

Als hydrothermal wird in diesem Zusammenhang nicht überkritisches Wasser bei Temperaturen oberhalb 100°C bezeichnet, das durch Druck größer oder gleich dem der Temperatur zugehörigen Dampfdruck im flüssigen Zustand vorliegt. Als überkritisch wird Wasser bei Temperaturen und Drücken höher oder gleich des kritischen Punktes bei 374,12°C und 22,1 MPa bezeichnet.

Überraschenderweise ist es gelungen, zusätzlich zur Spaltung der Si-C Bindungen die Si-O Bindungen in Siliconen durch hydrothermale Umsetzung zu spalten und die entstehenden Intermediate in einen Feststoff, ähnlich der Fällungskieselsäure umzuwandeln. Die hydrothermale Spaltung war bisher nur bei Si-C Bindungen bekannt.

Das Verfahren bietet die Möglichkeit, flüssige und feste Silicone in der Flüssigphase oder überkritischen Phase umzusetzen. Die Reaktionsmischung aus Wasser und Silicon/Siloxan liefert dabei ein hydrophobes, ungiftiges Feststoffgemisch, das im Nachgang entweder unproblematisch entsorgt, oder aufgrund der hydrophoben Eigenschaften als Füllmaterial verwendet werden kann. Dieses Feststoffgemisch kann auch der Rohsiliciumherstellung zugeführt werden, was eine Schließung des Stoffkreislaufs Silicium -> Methylchlorsilane -> Siloxan -> Feststoffgemisch -> Silicium bedeutet. Der im Feststoff enthaltene Kohlenstoff trägt zu einer Reduktion der zur Rohsiliciumgewinnung benötigten Menge an Kohle bei. Dieses Feststoffgemisch kann auch als Füllstoff für Zementmischungen verwendet werden.

Die Silicone enthalten vorzugsweise höchstens 1 Gew.-%, besonders bevorzugt höchstens 0,1 Gew.-%, insbesondere höchstens 0,01 Gew.-% an Halogenen, insbesondere Chlor. Organosiloxane enthaltende Zusammensetzungen können neben Organosiloxanen beispielsweise Füllstoffe, Katalysatoren, Bindemittel und Pigmente enthalten. Füllstoffe sind beispielsweise pyrogene und/oder gefällte Kieselsäure, Kreide und Quarz.

Das Verfahren kann diskontinuierlich oder kontinuierlich durchgeführt werden.

Das beim Verfahren anfallende Feststoffgemisch enthält vorzugsweise 25 bis 50 Gew.-%, besonders bevorzugt 30 bis 45 Gew.-%, insbesondere 31 bis 40 Gew.-% Silicium. Das Feststoffgemisch enthält vorzugsweise 0 bis 65 Gew.-%, besonders bevorzugt 8 bis 40 Gew.-%, insbesondere mindestens 15 bis 35 Gew.-% Kohlenstoff.

Der Kohlenstoff- und Sauerstoffanteil im Feststoff ist hierbei direkt abhängig von Reaktionstemperatur und der Zusammensetzung der Ausgangsstoffe. Höhere Temperaturen führen zu geringeren Kohlenstoff- und höheren Sauerstoffgehalten.

In einer besonderen Ausführungsform entsteht ein Gasgemisch. Das Gasgemisch enthält vorzugsweise Methan. Pro kg eingesetztem Silicon können bis zu 400 g Methan hergestellt werden. Vorzugsweise werden pro kg eingesetztem Silicon 10 bis 300 g Methan hergestellt.

In einer besonderen Ausführungsform entsteht beim Verfahren auch eine Flüssigphase. Die Flüssigphase enthält Wasser, Nebenprodukte der Reaktion und Verunreinigungen, die durch die Edukte eingetragen werden. Je nach Prozessführung kann ein Teil oder die gesamte Flüssigphase wieder als Edukt eingesetzt werden, beispielsweise um Abwasseranfall zu minimieren.

Neben Methan entstehen in veränderlichen Anteilen Kohlenstoffmonoxid, Kohlenstoffdioxid, höhere Kohlenwasserstoffe, Wasserstoff und Wasser. Die Zusammensetzung des Gasgemischs hängt von Druck und Temperatur ab. Mit höherer Temperatur unter höherem Druck kann der Anteil von Methan im Gasgemisch erhöht werden.

Die bevorzugte Temperatur im Verfahren beträgt 160°C bis 700°C, insbesondere 200°C bis 400°C und besonders bevorzugt 250°C - 390°C. Der bevorzugte Druck im Verfahren beträgt von 10 bis 400 bar, besonders bevorzugt 20 bis 320 bar, insbesondere 50 bis 280 bar, wobei als untere Grenze des Drucks jeweils der zur Reaktionstemperatur zugehörige Dampfdruck der Reaktionsmischung gilt. Die bevorzugte Verweilzeit im Verfahren beträgt 1 Minute bis 24 Stunden, besonders bevorzugt 5 Minuten bis 10 Stunden, insbesondere 10 Minuten bis 1 Stunde.

In einer bevorzugten Ausführungsform wird das Reaktionsgemisch nach Beendigung der Umsetzung auf atmosphärischen Druck entspannt und der entstehende Dampf zur Wärmerückgewinnung bzw. Vorwärmung der Edukte genutzt. Dadurch kann die Wärme zum großen Teil zurückgewonnen werden.

Die nachfolgenden Beispiele dienen zur weiteren Erläuterung der hier beschriebenen Erfindung.

Folgende analytische Methoden und Geräte werden zur Charakterisierung eingesetzt:
Die in den Beispielen angegebenen Elementargehalte werden mit einem energiedispersen Röntgenspektrometer (EDX) gemessen. Die EDX-Untersuchungen wurden mit einem Rasterelektronenmikroskop Zeiss Ultra 55 und einem energiedispersem Röntgenspektrometer Oxford X-Max 80N durchgeführt. Die Proben wurden vor der Untersuchung zur Verhinderung von Aufladungsphänomenen mit einer Safematic Compact Coating Unit 010/HV mit Kohlenstoff bedampft. Die Analyse der Gasphase erfolgt mittels Massenspektrometrie (ThermoStar^{™} GSD 320 T2 mit Iridiumkathode).

Folgende Materialien und Geräte werden bei der Durchführung experimenteller Beispiele eingesetzt:
Der in Beispiel 1 eingesetzte Autoklav besteht aus einem zylindrischen Unterteil (Becher) und einem Deckel mit mehreren Anschlüssen (beispielsweise für Gasabfuhr, Temperatur- und Druckmessung). Das Volumen des Autoklaven beträgt 594 ml. Der Autoklav wird elektrisch beheizt. Der eingesetzte Rührer ist ein vierblättriger Schrägblattrührer mit einem Durchmesser von 5 cm.

Das in Beispiel 1 eingesetzte Silicon ist nach Anleitung ausgehärtetes ELASTOSIL^{®} LR 7665 von WACKER CHEMIE AG, München. Das eingesetzte Wasser ist destilliertes Wasser.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

### Beispiel 1:

Ein Autoklav wird mit der Menge 30 g an klein geschnittenem (Würfel mit der Kantenlänge 0,5 cm) Silicon und 150 ml Wasser befüllt und verschlossen. Der Autoklav wird innerhalb 120 Minuten auf eine Temperatur von 350°C erhitzt, die Temperatur wird für 90 Minuten gehalten. Im Verlauf des Versuchs steigt der Druck auf 160 bar an. Innerhalb von 12 Stunden kühlt der Autoklav auf Raumtemperatur ab. Nach Abkühlung des Reaktors wird die Probe für die Gasanalyse entnommen. Anschließend wird der Gasraum des Autoklaven mit Stickstoff gespült und der Autoklav geöffnet.

Es wird eine klare Flüssigkeit und ein weißer oder leicht grauer Feststoff erhalten. Nach dem Abtrennen der Flüssigkeit und Trocknung des Feststoffs wird 21 g Feststoff erhalten.

Es wurden 3 Versuche durchgeführt. Die Ergebnisse der EDX-Analysen des Feststoffgemisches sind in Tabelle 1 aufgeführt. MW bedeutet Mittelwert

**Tabelle 1:**

| Versuch Nr. | 1 | 2 | 3 | Max | Min | MW |
|---|---|---|---|---|---|---|
| C (Gew.-%) | 23,96 | 34,09 | 29,64 | 34,09 | 23,96 | 29,23 |
| O (Gew.-%) | 41,35 | 32,64 | 38,46 | 41,35 | 32,64 | 37,48 |
| Na (Gew.-%) | 0, 00 | 0,21 | 0,29 | 0,29 | 0,00 | 0,17 |
| Si (Gew.-%) | 34,70 | 33,05 | 31,61 | 34,70 | 31,61 | 33,12 |
| Gesamt (Gew.-%) | 100,01 | 99, 99 | 100,00 | | | |

### Beispiel 2:

1000 kg feste Siliconabfälle, bestehend aus Siliconkautschuk in Gebinden aus Kunststoff, werden inklusive Gebinde zerkleinert und in einem Rührkessel der Größe 3 m³ zusammen mit 1500 kg Wasser bei 350 °C und 160 bar für 2 h umgesetzt. Es werden zwei Kessel parallel betrieben. Die Vorwärmung des einen Kessels wird durch Abkühlung des anderen Kessels mittels Wärmerückgewinnung realisiert, die restliche benötigte Wärme wird extern zugeführt. Die Produkte der Umsetzung sind eine Feststoff-Flüssig-Mischung und eine Gasphase. Die Feststoff-Flüssig-Mischung wird filtriert, der gewonnene Feststoff hat die Zusammensetzung aus Tabelle 1. Eventuell zugefügte Gebinde aus Kunststoff werden gemäß dem Stand des Wissens der hydrothermalen Karbonisierung zu kohlenstoffreichen Feststoffpartikeln umgesetzt. Die Flüssigphase enthält Nebenprodukte der Reaktion und kann, je nach Grad der Verunreinigung, zur erneuten Umsetzung von Siliconabfällen eingesetzt werden. Die Gasphase enthält neben Wasser flüchtige

Nebenprodukte zu denen auch, je nach Reaktionsführung und Ausgangsprodukten, Methan zählen kann.

Der Feststoff wird der Verwertung aus Beispiel 3 zugeführt.

### Beispiel 3:

Verwertung des Feststoffes aus Beispiel 1 und Beispiel 2 zur Herstellung von metallurgischem Silicium.

Der in den Beispielen 1 und 2 gewonnene Feststoff wird zu Pellets gepresst und als Edukt der Rohsiliciumgewinnung zugeführt. Das Silicium wird dabei zu Rohsilicium reduziert.

## Patentansprüche

1. Verfahren zur Umsetzung von Silicon mit hydrothermalem Wasser ab Temperaturen von 140°C oder überkritischem Wasser zu einem Feststoffgemisch, das Silicium enthält,
bei dem der Begriff Silicon oligomere oder polymere Organosiloxane umfasst, in denen Siliciumatome über Sauerstoffatome verbunden sind und in denen mindestens ein Teil der Siliciumatome einen oder mehrere organische Substituenten tragen, sowie Organosiloxane enthaltende Zusammensetzungen.

2. Verfahren nach einem oder mehreren der voranstehenden Ansprüche, bei dem die Silicone höchstens 1 Gew.-% an Halogenen enthalten.

3. Verfahren nach einem oder mehreren der voranstehenden Ansprüche, bei dem das Feststoffgemisch 25 bis 50 Gew.-% Silicium enthält.

4. Verfahren nach einem oder mehreren der voranstehenden Ansprüche, bei dem ein Methan enthaltendes Gasgemisch entsteht.

5. Verfahren nach einem oder mehreren der voranstehenden Ansprüche, bei dem die Temperatur 160°C bis 700°C beträgt.

6. Verfahren nach einem oder mehreren der voranstehenden Ansprüche, bei dem der Druck 10 bis 400 bar beträgt.

7. Verfahren nach einem oder mehreren der voranstehenden Ansprüche, bei dem das hergestellte Feststoffgemisch der Rohsiliciumherstellung zugeführt wird.

## Claims

1. Process for reacting silicone with hydrothermal water at temperatures from 140°C or supercritical water to give a solids mixture containing silicon,
wherein the term "silicone" encompasses oligomeric or polymeric organosiloxanes in which silicon atoms are bonded via oxygen atoms and in which at least some of the silicon atoms carry one or more organic substituents, and also compositions comprising organosiloxanes.

2. Process according to one or more of the preceding claims, wherein the silicones contain at most 1 wt% of halogens.

3. Process according to one or more of the preceding claims, wherein the solids mixture contains 25 to 50 wt% of silicon.

4. Process according to one or more of the preceding claims, wherein a methane-containing gas mixture is produced.

5. Process according to one or more of the preceding claims, wherein the temperature is 160°C to 700°C.

6. Process according to one or more of the preceding claims, wherein the pressure is 10 to 400 bar.

7. Process according to one or more of the preceding claims, wherein the solids mixture produced is supplied to raw silicon production.

## Revendications

1. Procédé pour la mise en réaction de silicone avec de l'eau hydrothermique à partir de températures de 140 °C ou de l'eau supercritique, conduisant à un mélange de solides qui contient du silicium, dans lequel le terme silicone comprend des organosiloxanes oligomères ou polymères, dans lesquels des atomes de silicium sont liés par l'intermédiaire d'atomes d'oxygène et dans lesquels au moins une partie des atomes de silicium portent un ou plusieurs substituants organiques, ainsi que des compositions contenant des organosiloxanes.

2. Procédé selon une ou plusieurs des revendications précédentes, dans lequel les silicones contiennent au maximum 1 % en poids d'halogènes.

3. Procédé selon une ou plusieurs des revendications précédentes, dans lequel le mélange de solides contient 25 à 50 % en poids de silicium.

4. Procédé selon une ou plusieurs des revendications précédentes, dans lequel il se forme un mélange de gaz contenant du méthane.

5. Procédé selon une ou plusieurs des revendications précédentes, dans lequel la température est de 160 °C à 700 °C.

6. Procédé selon une ou plusieurs des revendications précédentes, dans lequel la pression est de 10 à 400 bars.

7. Procédé selon une ou plusieurs des revendications précédentes, dans lequel le mélange de solides produit est envoyé à la production de silicium brut.
